Europäisches Patentamt

European Patent Office

Office européen des brevets

(19)

(11) Veröffentlichungsnummer: **0 014 366**
**B1**

(12) **EUROPÄISCHE PATENTSCHRIFT**

(45) Veröffentlichungstag der Patentschrift:
16.09.81

(21) Anmeldenummer: 80100275.9

(22) Anmeldetag: 21.01.80

(51) Int. Cl.³: **C 07 C 143/55**, C 07 C 139/00,
C 07 C 139/14

(54) Verfahren zur Herstellung des Magnesiumsalzes von 2-Nitronaphthalin-4,8-disulfonsäure (Nitro-Armstrong-Säure).

(30) Priorität: 01.02.79 DE 2903849

(43) Veröffentlichungstag der Anmeldung:
20.08.80 Patentblatt 80/17

(45) Bekanntmachung des Hinweises auf die Patenterteilung:
16.09.81 Patentblatt 81/37

(84) Benannte Vertragsstaaten:
BE CH DE FR GB IT

(56) Entgegenhaltungen:
FR-A-2 413 367
US-A-1 756 537
US-A-1 836 204
US-A-2 191 820
US-A-2 875 242
CHEMICAL ABSTRACTS, Band 60, Nr. 6, 16. März 1964,
Zusammenfassung Nr. 6802 e–f, Columbus, Ohio, US

(73) Patentinhaber: **BAYER AG, Zentralbereich Patente,
Marken und Lizenzen, D-5090 Leverkusen 1, Bayerwerk
(DE)**

(72) Erfinder: **Behre, Horst, Dr., Im Heilsiefen 4,
D-5068 Odenthal (DE)**
Erfinder: **Blank, Heinz Ulrich, Dr., Am Geusfelde 35,
D-5068 Odenthal (DE)**
Erfinder: **Pelster, Heinrich, Dr., Auf dem Heidchen 23,
D-5068 Odenthal (DE)**
Erfinder: **Schenk, Wolfgang, Dr.,
Odenthalerstrasse 62/64, D-5090 Leverkusen (DE)**
Erfinder: **Steffan, Guido, Dr., Im Herzogenfeld 52,
D-5068 Odenthal (DE)**
Erfinder: **Vogel, Axel, Dr., In der Hildscheid 5,
D-5068 Odenthal (DE)**

ACTORUM AG.

## Verfahren zur Herstellung des Magnesiumsalzes von 2-Nitronaphthalin-4,8-disulfonsäure (Nitro-Armstrong-Säure)

Die vorliegende Erfindung betrifft ein Verfahren zur Herstellung des Magnesiumsalzes von 2-Nitronaphthalin-4,8-disulfonsäure (Nitro-Armstrong-Säure, im folgenden auch als Nitro-AS bezeichnet) durch Nitrierung eines Produktgemisches, das durch Umsetzung von Naphthalin mit Schwefeltrioxid in einem inerten Lösungsmittel erhalten wurde, und Versetzen des verdünnten Nitriergemisches mit einer Magnesiumionen abgebenden Verbindung.

Aus dem US-Patent 2 191 820 ist bekannt, dass man Nitro-AS herstellen kann, indem man zunächst reine Naphthalin-1,5-disulfonsäure und 60%iges Oleum unterhalb von 40 °C in Monohydrat (100%ige $H_2SO_4$) einträgt. Die Mischung wird dann unterhalb 30 °C langsam mit einer wasserfreien Mischung aus Schwefelsäure und Salpetersäure ($HNO_3$-Gehalt 40%) versetzt und 6 bis 7 Stunden bei 30 bis 35 °C nachgerührt. Anschliessend wird das Nitriergemisch mit Wasser verdünnt und die Nitro-AS nach Zugabe von Magnesiumoxid als Magnesiumsalz isoliert.

Es wurde nun ein Verfahren zur Herstellung des Magnesiumsalzes von 2-Nitronaphthalin-4,8-disulfonsäure durch Nitrierung von 1,5-disulfiertem Naphthalin, Abtrennung von 2-Nitronaphthalin-4,8-disulfonsäure als Magnesiumsalz und Wäsche des Magnesiumsalzes gefunden, das dadurch gekennzeichnet ist, dass man

a) von 1,5-disulfiertem Naphthalin in Form eines Gemisches ausgeht, das erhalten worden ist durch Zusammengabe von Naphthalin und $SO_3$ in Gegenwart eines inerten Lösungsmittels bei Temperaturen im Bereich $-40$ bis $+20$ °C, wobei das Verhältnis von zugegebenem $SO_3$ zu zugegebenem Naphthalin während der gesamten Zugabe im Bereich von 2,5 bis 5 Mol $SO_3$ pro Mol Naphthalin gelegen hat,

b) dieses Gemisch, gegebenenfalls nach Abtrennung des inerten Lösungsmittels, in wasserfreier Schwefelsäure mit einer Salpetersäure, Schwefelsäure und mindestens 0,7 Mol $SO_3$ pro Mol Salpetersäure enthaltenden Mischsäure bei Temperaturen im Bereich von 10 bis 60 °C und im Verlaufe von 1 bis 10 Stunden nitriert, wobei man das Gemisch und die Mischsäure simultan oder wechselweise in Portionen mit vorgelegter Schwefelsäure oder dem sich bildenden Reaktionsgemisch vereinigt,

c) danach zunächst Wasser und dann in Abwesenheit des inerten Lösungsmittels bei 60 °C bis 120 °C soviel einer Magnesiumionen abgebenden Verbindung zugibt, dass pro Mol 2-Nitronaphthalin-4,8-disulfonsäure 1,0 bis 1,3 Mol Magnesiumionen zugegeben werden und eine Schwefelsäurekonzentration im Bereich von 40 bis 60 Gew.-% (bezogen auf Schwefelsäure und Wasser) eingehalten wird,

d) das ausgefallene Magnesiumsalz der 2-Nitronaphthalin-4,8-disulfonsäure bei einer Temperatur im Bereich von 30 bis 80 °C abtrennt und anschliessend mit maximal 60 gew.-%iger Schwefelsäure und/oder mit Wasser wäscht.

Das Einsatzprodukt für das erfindungsgemässe Verfahren kann vorzugsweise erhalten werden, indem man Naphthalin und $SO_3$ jeweils getrennt in einem inerten Lösungsmittel, vorzugsweise in einem aliphatischen Chlorkohlenwasserstoff mit 1 bis 3 C-Atomen, insbesondere Dichlormethan, löst und beide Lösungen bei ca. $-10$ bis $-5$ °C vereinigt, wobei während der gesamten Zugabe ein Molverhältnis von zugegebenem $SO_3$ zu zugegebenem Naphthalin im Bereich von 2,5 bis 3,6, vorzugsweise im Bereich von 3,0 bis 3,2, eingehalten wird. Dabei bilden sich ein Sulfierprodukt von Naphthalin, welches sich in fester Form abscheidet und $H_2SO_4$ und gegebenenfalls $SO_3$ enthält. Das Sulfierprodukt fällt als Suspension in dem verwendeten Lösungsmittel an.

Dieses Sulfierprodukt des Naphthalins, im folgenden auch als Armstrongsäureanhydrid oder AS-AN bezeichnet, ist ein Gemenge oligomerer Anhydride von Naphthalin-di- und -trisulfonsäuren mit $H_2So_4$ und gegebenenfalls $SO_3$. Wenn man die im AS-AN vorhandenen sulfierten Naphthalineinheiten formal betrachtet als Naphthalinsulfonsäuren und die $H_2SO_4$ als $SO_3$ auffasst, so kann das AS-AN beispielsweise 69 bis 88 Gew.-% Naphthalin-di- und -trisulfonsäuren und 31 bis 12 Gew.-% $SO_3$ enthalten und enthält im allgemeinen, bezogen auf die Gesamtmenge an Naphthalinsulfonsäuren, über 65 Gew.-% Naphthalin-1,5-disulfonsäure.

Bevorzugt werden in das erfindungsgemässe Verfahren Gemische eingesetzt, die, formal betrachtet, 70 bis 82 Gew.-% Naphthalin-1,5-disulfonsäure, bezogen auf die Gesamtmenge an Naphthalinsulfonsäuren, enthalten. Ein besonders bevorzugtes AS-AN enthält:

75 bis 80 Gew.-% Naphthalin-1,5-disulfonsäure.
0 bis 3 Gew.-% Naphthalin-1,3-disulfonsäure
5 bis 15 Gew.-% Naphthalin-1,6-disulfonsäure
2 bis 5 Gew.-% Naphthalin-1,7-disulfonsäure
0 bis 5 Gew.-% Naphthalin-1,3,5-trisulfonsäure
0 bis 5 Gew.-% Naphthalin-1,3,6-trisulfonsäure
0 bis 1 Gew.-% Naphthalin-1,3,7-trisulfonsäure
0 bis 1 Gew.-% Naphthalin-1-sulfonsäure.
jeweils bezogen auf die Summe aller Naphthalinderivate und gerechnet als freie Sulfonsäure.

Darüber hinaus kann das AS-AN in sehr geringen Mengen weitere, nicht näher identifizierte Dinaphthylsulfone und Dinaphthylsulfon-sulfonsäuren enthalten.

Die Stufe b) des erfindungsgemässen Verfahrens kann in Anwesenheit oder Abwesenheit des im Einsatzprodukt vorhandenen Lösungsmittels durchgeführt werden. Wesentlich für die Durchführung der Stufe b) ist, dass man die Nitrierung in wasserfreier Schwefelsäure durchführt, beispielsweise in 100%iger Schwefelsäure oder in Schwefelsäure, die 0 bis 10 Gew.-% freies $SO_3$ enthält. Wesentlich ist ferner, dass man für die Nitrierung eine Mischsäure verwendet, die Salpetersäure, Schwefelsäure und mindestens 0,7 Mol SO$_3$ pro Mol Salpetersäure enthält. Vorzugsweise enthält die Mischsäure 0,8 bis 1,2 Mol SO$_3$ pro Mol Salpetersäure. Solche Mischsäuren können durch Vermischung von Salpetersäuren und Oleum hergestellt werden. Der Anteil Schwefelsäure in der Mischsäure kann beliebig gewählt werden.

Die simultane Vereinigung des Gemisches mit Mischsäure in vorgelegter Schwefelsäure oder dem sich bildenden Reaktionsgemisch (Nitriergemisch) oder deren wechselseitige Zugabe in Portionen zum Nitriergemisch hat den Vorteil, dass man die Nitrierung mit besonders hoher Raum-Zeit-Ausbeute und mit besonders geringem Verbrauch an Schwefelsäure durchführen kann.

Pro Mol Naphthalin in Form des AS-AN können beispielsweise 1,0 bis 1,5 Mol Salpetersäure, vorzugsweise 1,1 bis 1,3 Mol Salpetersäure, in Form der Mischsäure eingesetzt werden. Die Temperatur in Stufe b) liegt im Bereich von 10 bis 60 °C, vorzugsweise im Bereich 20 bis 50 °C, insbesondere im Bereich 35 bis 45 °C.

Die Durchführung der Stufe b) des erfindungsgemässen Verfahrens kann in verschiedenen Varianten erfolgen. Zwei bevorzugte Varianten sind im folgenden detailliert angegeben.

Variante 1:

Aus dem Einsatzprodukt wird zunächst das Lösungsmittel entfernt. Dies kann z.B. durch Filtration mit anschliessender Trocknung oder nur durch Trocknung erfolgen. Das so abgetrennte Lösungsmittel kann erneut bei der Sulfierung von Naphthalin verwendet werden. Das trockene Gemisch (AS-AN) und die Mischsäure werden dann simultan in vorgelegte wasserfreie Schwefelsäure eindosiert. Beispielsweise kann man 1 bis 10 Mol Monohydrat (= 100%ige H$_2$SO$_4$), vorzugsweise 4 bis 6 Mol Monohydrat pro Mol Naphthalin in Form von AS-AN vorlegen. Die Eindosierung muss nicht streng simultan erfolgen, man kann auch wechselweise kleine Portionen AS-AN und Mischsäure in die wasserfreie Schwefelsäure eindosieren.

Die Dosierarbeiten für AS-AN und Mischsäure betragen 1 bis 10 Stunden. Die Dosierzeiten hängen im wesentlichen von der Reaktionstemperatur und der SO$_3$-Konzentration im Reaktionsgemisch ab. Bei relativ hohen Temperaturen und relativ niedrigen SO$_3$-Konzentrationen kann eine relativ kurze Dosierzeit, bei relativ niedrigen Temperaturen und relativ hohen SO$_3$-Konzentrationen eine längere Dosierzeit gewählt werden. Vorzugsweise liegt die Dosierzeit im Bereich zwischen 1 und 6 Stunden. Die Dosiergeschwindigkeit wird vorzugsweise so gewählt, dass sie etwa der Nitriergeschwindigkeit entspricht. Dann bleibt das Reaktionsgemisch zu jedem Zeitpunkt gut rühr- und pumpbar. Nach beendeter Dosierung lässt man das Reaktionsgemisch im Verlaufe von bis zu 3 Stunden bei 10 bis 60 °C ausreagieren. Vorzugsweise beträgt die Nachreaktionszeit 1 bis 2 Stunden bei 35 bis 45 °C.

Variante 2:

Die Nitrierung wird, wie in Variante 1 beschrieben, durchgeführt, jedoch wird das Lösungsmittel vor der Nitrierung nicht entfernt, sondern erst nach Ende der Nitrierung oder während der Zugabe von Wasser zum Nitriergemisch, wobei die Entfernung des Lösungsmittels z.B. durch Phasentrennung und/oder Destillation erfolgen kann. Auch hier kann das abgetrennte Lösungsmittel, gegebenenfalls nach Trocknung, erneut bei der Sulfierung von Naphthalin verwendet werden.

Besonders bevorzugt wird gemäss der Variante 1 gearbeitet.

Das erfindungsgemässe Verfahren kann in beiden Varianten auch kontinuierlich durchgeführt werden.

Unter den erfindungsgemässen Nitrierbedingungen werden die im AS-AN neben den 1,5-disulfierten Naphthalineinheiten hauptsächlich vorhandenen 1,6-disulfierten Naphthalineinheiten im wesentlichen zu 2-Nitro-naphthalin-4,7-disulfonsäure umgesetzt. Diese hat ähnliche Löslichkeitseigenschaften wie die Nitro-AS. Bei der Weiterverarbeitung der Nitro-AS zu C-Säure ist es jedoch erforderlich, dieses Nebenprodukt vorher zu entfernen, da die C-Säure sehr gut löslich ist und normalerweise durch Eindampfen der Reaktionslösung zur Herstellung von C-Säure gewonnen wird.

Ein wesentliches Merkmal des erfindungsgemässen Verfahrens ist deshalb die Isolierung von praktisch reinem Magnesiumsalz der 2-Nitronaphthalin-4,8-disulfonsäure, das nur ganz geringe Mengen 2-Nitronaphthalin-4,7-disulfonsäure enthält. Hierzu wird das Nitriergemisch zunächst mit Wasser verdünnt, vorzugsweise indem man das Nitriergemisch in vorgelegtes Wasser eingibt. Anschliessend wird eine Magnesiumionen abgebende Verbindung zugegeben, wobei die in Anspruch 1 genannten Bedingungen eingehalten werden. Als Magnesiumionen abgebende Verbindungen kommen beispielsweise Magnesiumsalze, wie Magnesiumsulfat und Magnesiumcarbonat, und Magnesiumoxid in Frage. Diese Verbindungen können z.B. gelöst in Wasser oder im Gemisch mit Wasser angewendet werden. Vorzugsweise wird eine wässrige Magnesiumsulfatlösung verwendet.

Vorzugsweise erfolgt die Fällung des Magnesiumsalzes der Nitro-AS, d.h. die Zugabe der Magnesiumionen abgebenden Verbindungen, bei 75 bis 100 °C, und die Abtrennung des Magnesiumsalzes der Nitro-AS bei 40 bis 70 °C. Die Abtrennung des Magnesiumsalzes der Nitro-AS kann z.B. durch Filtration erfolgen. Vorzugsweise werden dabei 1,15 bis 1,25 Mol Magnesiumsulfat pro Mol 2-Nitronaphthalin-4,8-disulfonsäure eingesetzt. Eine besonders günstige Ausführungsform für die Fällung des Magnesiumsalzes der Nitro-AS besteht darin, dass man die verdünnte und von nitrosen Gasen befreite Nitro-AS-Lösung und eine wässrige Magnesiumsulfatlösung simultan in einen Fällungskessel eindüst.

Zur Filtration des Magnesiumsalzes der Nitro-AS kann insbesondere ein Pressfilterautomat verwendet werden. Dadurch lassen sich Produkte mit einem geringen Restfeuchtegehalt von nur ca. 30% und somit mit sehr geringen Gehalten an Schwefelsäure erhalten.

Die Isolierung des Magnesiumsalzes der Nitro-AS durch Filtration und Wäsche bereitet nach den literaturbekannten Verfahren erhebliche Schwierigkeiten infolge der schlecht ausgebildeten Kristalle des Magnesiumsalzes der Nitro-AS und deren Neigung zur Ausbildung thixotroper Pasten mit hohen Restfeuchten, die eine entsprechende Verunreinigung bedingen. Als technisch gangbarer Weg hat sich bisher lediglich die Wäsche des Filterkuchens mit wässriger Natriumchloridlösung bewährt, was aber eine Verunreinigung des Magnesiumsalzes der Nitro-AS mit Natriumchlorid zur Folge hat, das sich bei der Weiterverarbeitung der Nitro-AS zur C-Säure in der C-Säure wiederfindet.

Die Filtration und Wäsche des nach dem erfindungsgemässen Verfahrens erhaltenen Magnesiumsalzes der Nitro-AS erfolgt vorzugsweise auf einem Pressfilterautomaten. Überraschenderweise erhält man auf diesem Filterapparat einen Filterkuchen, der die literaturbekannte schlechte Filtrierbarkeit nicht aufweist und damit technisch wesentlich besser handhabbar ist. Insbesondere wird auch eine geringe Restfeuchte erreicht.

Das Magnesiumsalz der Nitro-AS kann zur Entfernung der Nebenprodukte der Nitrierung mit maximal 60%iger Schwefelsäure, vorzugsweise mit 40 bis 60%iger Schwefelsäure gewaschen werden. Um das Magnesiumsalz der Nitro-AS weitgehend schwefelsäurefrei zu erhalten ist eine Wäsche mit Wasser vorteilhaft. Die Waschmittelmenge an Schwefelsäure und/oder Wasser kann beispielsweise jeweils 0,5 bis 3 kg pro Mol Magnesiumsalz der Nitro-AS betragen, vorzugsweise jeweils 1 bis 2 kg pro Mol Magnesiumsalz der Nitro-AS.

Das erfindungsgemässe Verfahren weist eine Reihe von Vorteilen auf, die überrraschend sind.

Das bekannte Herstellungsverfahren für das Magnesiumsalz der Nitro-AS entsprechend dem US-Patent 2 191 820 geht von reiner Naphthalin-1,5-disulfonsäure aus. Da bei der Disulfierung von Naphthalin stets in nicht unerheblichem Umfang auch Isomere der Naphthalin-1,5-sulfonsäure und Naphthalintrisulfonsäuren entstehen, ist bei dem Verfahren des US-Patentes 2 191 820 eine aufwendige und umständliche Zwischenisolierung von Naphthalin-1,5-disulfonsäure nötig. Eine derartige Zwischenisolierung entfällt beim erfindungsgemässen Verfahren, trotzdem wird das Magnesiumsalz der Nitro-AS in hoher Reinheit erhalten.

Im US-Patent 2 191 820 ist angegeben, dass die Nitrierung unter wasserfreien Bedingungen durchzuführen ist, um die Bildung von 1-Nitronaphthalin-4,8-disulfonsäure zu unterdrücken. Da Naphthalin-1,5-disulfonsäure als Tetrahydrat kristallisiert (s. Ullmann's Enzyklopädie der technischen Chemie, dritte Auflage, 12. Band, Seite 595) müssen dem Verfahren gemäss dem US-Patent 2 191 820 erhebliche Mengen Oleum zugeführt werden, um durch Bindung des Kristallwassers die Nitrierung in wasserfreiem Medium zu ermöglichen. Im erfindungsgemässen Verfahren wird Naphthalin-1,5-disulfonsäure in Form des wasserfreien AS-AN eingesetzt und deshalb wesentlich weniger Oleum bzw. $SO_3$ benötigt, um das Nitriergemisch wasserfrei zu halten.

Es ist als ausgesprochen überraschend anzusehen, dass es unter den erfindungsgemässen Bedingungen gelingt, das Magnesiumsalz der Nitro-AS in einer Form abzuscheiden, die gut filtrierbar und waschbar ist und die eine ausgezeichnete Abtrennung der Isomeren gestattet. Aus dem US-Patent 1 756 537 (1930) ist zwar bekannt, dass man aus Nitriergemischen, die bei der Nietrierung von Naphthalin-1,5-disulfonsäure oder deren Mischungen mit Naphthalin-1,6-disulfonsäure anfallen, durch Zugabe von Magnesiumsalz bei Temperaturen zwischen 0 und 100 °C und Filtration bei 0 bis 10 °C das Magnesiumsalz der Nitro-AS isolieren kann. In dem nachveröffentlichen SU-Erfinderschein 154 261 (1963) wird jedoch beschrieben, dass das Magnesiumsalz der Nitro-AS in Form kleiner Kristalle anfällt, die schlecht filtrierbar und waschbar sind und aus denen die Abtrennung von Isomeren erschwert ist. Im SU-Erfinderschein 164 261 wird deshalb vorgeschlagen, die Abtrennung von Nitro-AS aus Nitriergemischen in Form des Ammoniumsalzes vorzunehmen, weil dabei grobe, leicht filtrierbare und waschbare Kristalle anfallen.

Die mit dem erfindungsgemässen Verfahren erzielbaren Ausbeuten sind hoch. Bezogen auf in die Sulfierung eingesetztes Naphthalin kann eine Ausbeute an isoliertem Magnesiumsalz der Nitro-AS von ca. 70% erhalten werden. Durch die konzentrierte Arbeitsweise ist auch die Raum/Zeit-Ausbeute sehr günstig. Die Reinheit des erfindungsgemäss hergestellten Magnesiumsalzes der Nitro-AS ist hoch, obwohl keine reine Naphthalin-1,5-disulfonsäure in die Nitrierung eingesetzt wird und bekanntermassen bei der Nitrierung eine Reihe von unerwünschten Nebenprodukten entstehen (s. US-Patent 1 756 537). Der Verbrauch von Hilfsstoffen, wie Schwefelsäure und Oleum, ist gegenüber bekannten Verfahren stark vermindert. Es fallen deshalb auch nach der Isolierung des Magnesiumsalzes der Nitro-AS wesentlich geringere Mengen verdünnter Säuren an. Ein weiterer Vorteil besteht darin, dass nach dem erfindungsgemässen Verfahren eine praktisch von Schwefelsäure freie und salzfreie Nitro-AS erhalten werden kann und damit bei deren Weiterverarbeitung zu C-Säure auch salzarme C-Säure.

2-Nitronaphthalin-4,8-disulfonsäure bzw. das Magnesiumsalz davon ist eine wichtige Zwischenverbindung zur Herstellung der entsprechenden 2-Naphthylamin-4,8-disulfonsäure (C-Säure). C-Säure wird als Diazotierungskomponente für Baumwollfarbstoffe und als Zwischenprodukt zur Herstellung von 2-Naphthol-4,6-disulfonsäure verwendet (s. Ullmann's Enzyklopädie der technischen Chemie, 3. Auflage, 12. Band, Seite 629).

Beispiel 1

In einem 2-ltr.-Vierhals-Kolben, ausgestattet mit einer Dosierschnecke, einem Dosiertropftrichter, Innenthermometer und Säbelrührer werden 500 g 100 Gew.-%ige $H_2SO_4$ bei 40 °C vorgelegt. Bei 40 °C werden dann über die Dosierschnecke im Verlaufe von 5 Stunden unter Rühren 390 g des Produktes zugegeben, das wie folgt erhalten wurde:

128 g Naphthalin (1,0 Mol) wurden in 640 g Dichlormethan gelöst, gleichzeitig 264 g flüssiges $SO_3$ in 1025 g Dichlormethan. Beide Lösungen wurden simultan in 100 g vorgelegtes Dichlormethan so einlaufen gelassen, dass die Temperatur zwischen −5 und −10 °C gehalten werden konnte. Nach beendeter Zusammengabe wurde die Reaktionsmischung 1,5 Stunden bei −5 bis −10 °C gehalten und anschliessend im Vakuum zur Trockene eingedampft. Das so erhaltene Produkt hatte formal betrachtet folgende Zusammensetzung:

55,6 Gew.-% Naphthalin-1,5-Disulfonsäure
 0,2 Gew.-% Naphthalin-1,3-Disulfonsäure
 5,9 Gew.-% Naphthalin-1,6-Disulfonsäure
 2,3 Gew.-% Naphthalin-1,7-Disulfonsäure
 2,7 Gew.-% Naphthalin-1,3,5-Trisulfonsäure
 5,9 Gew.-% Naphthalin-1,3,6-Trisulfonsäure
 0,8 Gew.-% Naphthalin-1,3,7-Trisulfonsäure
26,0 Gew.-% $SO_3$.

Gleichzeitig werden über den Dosiertropftrichter 216 g einer Mischsäure der Zusammensetzung
32 Gew.-% $HNO_3$ (entspricht 1,1 Mol $HNO_3$),
41 Gew.-% $SO_3$,
27 Gew.-% $H_2SO_4$
simultan eindosiert. Nach Beendigung der Zugabe wird die Reaktionsmischung 2 Stunden bei 40 °C nachgerührt.

In einem Fällungsgefäss werden 625 g Wasser vorgelegt. Unter Rühren und Kühlen wird dann die Nitriermischung in dem Masse einlaufen gelassen, dass eine Temperatur von 85 °C nicht überschritten wird. Dann wird innerhalb 30 Minuten eine Lösung von 96,3 g Magnesiumsulfat (0,8 Mol) in 200 g Wasser bei 85 °C einlaufen gelassen. Die Reaktionsmischung wird unter Rühren innerhalb von 30 bis 60 Minuten auf 60 °C abgekühlt und das Produkt bei 60 °C abfiltriert. Das Produkt wird mit insgesamt 1000 g 50 Gew.-%iger $H_2SO_4$ gewaschen. Der Filterkuchen wird danach gut abgepresst und mit 500 g kaltem Wasser gewaschen. Die Waschlauge wird gesondert aufgefangen und bei der folgenden Partie im Fällungsgefäss anstelle von Wasser vorgelegt.

Bei dem so isolierten Magnesiumsalz der 2-Nitronaphthalin-4,8-Disulfonsäure (Nitro-Armstrong-Säure) beträgt die Ausbeute 68% bezogen auf eingesetztes Naphthalin bzw. 88% bezogen auf Naphthalin-1,5-Disulfonsäure in Form des eingesetzten Sulfierungsproduktes. Die Qualität des isolierten Produktes wurde durch Hochdruckflüssigkeitschromatographie wie folgt ermittelt:
2-Nitronaphthalin-4,8-disulfonsäure: 99,8 relativ
2-Nitronaphthalin-4,7-disulfonsäure: 0,1–0,2% relativ
Nitrierungsprodukte aus den übrigen Naphthalindi- und tri-sulfonsäuren konnten in dem isolierten Produkt nicht nachgewiesen werden.

Beispiel 2:

Eine wie in Beispiel 1 durchgeführte Reaktion, bei der ein Sulfierungsprodukt eingesetzt wurde, das wie in Beispiel 1 erhalten wurde, bei dem jedoch 240 g $SO_3$ in 930 g Dichloremthan gelöst wurde, und das formal betrachtet folgende Zusammensetzung aufwies:
60,6 Gew.-% Naphthalin-1,5-disulfonsäure
 9,4 Gew.-% Naphthalin-1,6-disulfonsäure
 9,0 Gew.-% weitere Naphthalin-disulfonsäuren
            und Naphthalin-trisulfonsäuren
20,8 Gew.-% $SO_3$
ergab das Magnesiumsalz von 2-Nitronaphthalin-4,8-disulfonsäure in gleicher Ausbeute und Reinheit wie in Beispiel 1.

Beispiele 3a bis 3l:

Es wurde wie in Beispiel 1 verfahren, jedoch wurden folgende Reaktionsparameter bei der Nitrierungsreaktion variiert:
1. Mol-Verhältnis vorgelegte 100%ige Schwefelsäure zu Naphthalin-Sulfierungsprodukt.
2. Mol-Verhältnis Salpetersäure zu Naphthalin-Sulfierungsprodukt.
3. Temperatur
4. Reaktionszeit
Die Zusammensetzung der Nitriergemische wurde mittels Hochdruckflüssigkeitschromatographie ermittelt. Die Ergebnisse sind in Tabelle 1 zusammengefasst.

Tabelle 1

| Beispiel | Einsatz | | Reaktionsbedingungen | | | Gehalt des Nitriergemisches bestimmt durch Hochdruckflüssigkeitschromatographie (Mol-% bezogen auf eingesetztes Naphthalin) | | | Ausbeute an Mg-Salz von 2-Nitronaphthalin-4,8-disulfonsäure (bezogen auf Naphthalin) Mol-% |
|---|---|---|---|---|---|---|---|---|---|
| | Molverhältnis 100%ige $H_SO_4$ zu Naphthalin (in Form des Sulfierproduktes) | Molverhältnis $HNO_3$ (als Mischsäure) zu Naphthalin (in Form des Sulfierproduktes) | Temperatur (h) | Dosierzeit (h) | Nachrührzeit (h) | 2-Nitronaphthalin-4,8-disulfonsäure | Naphthalin-1,5-disulfonsäure | 2-Nitronaphthalin-4,7-disulfonsäure | |
| 3 a | 5,5:1 | 1,1:1 | 35 | 5 | 2,5 | 69 | 0,8 | 4,2 | 68 |
| 3 b | 4,5:1 | 1,1:1 | 35 | 5 | 2,5 | 69 | 1,2 | 4,1 | 68 |
| 3 c | 3,5:1 | 1,1:1 | 35 | 5 | 2,5 | 67 | 2,4 | 4,1 | 66 |
| 3 d | 2,5:1 | 1,1:1 | 35 | 5 | 2,5 | 62 | 7,5 | 3,9 | 61 |
| 3 e | 4,5:1 | 1,1:1 | 20 | 6 | 4 | 68 | 2,0 | 4,3 | 67 |
| 3 f | 4,5:1 | 1,1:1 | 50 | 5 | 1 | 64 | 4,2 | 3,5 | 63 |
| 3 g | 5,0:1 | 1,15:1 | 40 | 3 | 3 | 69 | 1,5 | 4,0 | 67 |
| 3 h | 5,0:1 | 1,15:1 | 40 | 4 | 2 | 69 | 1,0 | 4,0 | 68 |
| 3 i | 5,0:1 | 1,2:1 | 45 | 4 | 2 | 68 | 1,8 | 4,0 | 67 |
| 3 k | 5,0:1 | 1,0:1 | 30 | 5 | 4 | 65 | 6,0 | 4,3 | 64 |
| 3 l | 5,0:1 | 1,3:1 | 30 | 5 | 4 | 67 | 3,0 | 2,5 | 67 |

In keinem Fall konnte im Nitriergemisch 1-Nitronaphthalin-4,8-disulfonsäure nachgewiesen werden.

Beispiel 4:

Es wurde wie in Beispiel 1 verfahren, wobei aus dem Sulfierungsprodukt von Naphthalin das Dichlormethan jedoch nicht entfernt, sondern dieses in Form einer 30 Gew.-%igen Suspension in Dichlormethan eingesetzt wurde. Das ergab eine Ausbeute des Magnesiumsalzes von 2-Nitronaphthalin-4,8-disulfonsäure von 67% (bezogen auf Naphthalin) bzw. von 87% (bezogen auf Naphthalin-1,5-disulfonsäure in Form des eingesetzten Sulfierungsproduktes). Das Dichlormethan wurde während der Zugabe des Nitrierbgemisches in die Wasservorlage aus dem Fällungsgefäss destillativ entfernt.

Beispiel 5a – 5k:

Es wurde wie in Beispiel 1 verfahren, jedoch wurden folgende Reaktionsparameter bei der Isolierung des Magnesiumsalzes von 2-Nitronaphthalin-4,8-disulfonsäure variiert:

1. $H_2SO_4$-Konzentration
2. Mol-Verhältnis Magnesiumsulfat zu Naphthalin (in Form des in die Nitrierung eingesetzten Naphthalin-Sulfierungsproduktes).
3. Isolier-Temperatur
4. Kristallisationstemperatur.

Die Qualität und Ausbeute der isolierten Produkte wurde mittels Hochdruckflüssigkeitschromatographie ermittelt. Die Ergebnisse sind in Tabelle 2 zusammengefasst.

Tabelle 2

| Beispiel | Isolierbedingungen | | | | Gehalt an 2-Nitronaphthalin-4,8-disulfonsäure im Nitriergemisch bestimmt durch HFC (Mol-%) | Ausbeute an isoliertem Magnesiumsalz der 2-Nitronaphthalin-4,8-disulfonsäure, bezogen auf Naphthalin (Mol-%) | Qualität des isolierten Magnesiumsalzes der 2-Nitronaphthalin-4,8-disulfonsäure | |
|---|---|---|---|---|---|---|---|---|
| | $H_2SO_4$-Konzentration bezogen auf $H_2SO_4$ plus Wasser (Gew.-%) | Molverhältnis $MgSO_4$ zu Naphthalin | Isoliertemperatur (°C) | Kristallisiertemperatur (°C) | | | 2-Nitronaphthalin-4,8-disulfonsäure (relative Gew.-%) | 2-Nitronaphthalin-4,7-disulfonsäure (relative Gew.-%) |
| 5 a | 60 | 0,8:1 | 60 | 85 | 69 | 67 | 99.8 | 0,1–0,2 |
| 5 b | 50 | 0,8:1 | 60 | 85 | 69 | 68 | 99.8 | 0,1–0,2 |
| 5 c | 40 | 0,8:1 | 60 | 85 | 69 | 67 | 99.8 | 0,1–0,2 |
| 5 d +) | 20 | 0,8:1 | 20 | 85 | 69 | 64 | 99.6 | 0,2–0,4 |
| 5 e+) | 50 | 0,8:1 | 20 | 85 | 69 | 68 | 97.0 | 2,5–3,0 |
| 5 f | 50 | 0,8:1 | 50 | 85 | 69 | 68 | 99.8 | 0,1–0,2 |
| 5 g | 50 | 0,8:1 | 70 | 85 | 69 | 67 | 99.8 | 0,1–0,2 |
| 5 h | 50 | 0,7:1 | 60 | 85 | 69 | 63 | 99.8 | 0,1–0,2 |
| 5 i | 50 | 1,0:1 | 60 | 85 | 69 | 68 | 98.5 | 1,0–1,5 |
| 5 k | 50 | 0,8:1 | 60 | 100 | 69 | 68 | 99.8 | 0,1–0,2 |

+) Diese Versuche dienen zum Vergleich; die Isolierbedingungen liegen ausserhalb der Ansprüche

**Beispiel 6:**

250 kg 100 Gew.-%ige $H_2SO_4$ werden in einem 0,5 m³-Rührwerkkessel vorgelegt. Im Verlaufe von 4 Stunden werden bei 40 °C 191 kg eines Naphthalin-Sulfierproduktes zugegeben, das wie folgt erhalten wurde:

In einem Reaktionskessel wurden unter Rühren eine Lösung von 64 kg Naphthalin in 320 kg Dichlormethan und eine Lösung von 128 kg $SO_3$ in 512 kg Dichlormethan im Verlauf von 1,5 Stunden bei einer Temperatur von − 6 °C gleichzeitig eindosiert. Nach einer Nachreaktionszeit von 1 Stunde wurde das Dichlormethan im Vakuum bei −6 bis +20 °C bis zur Trockene abdestilliert.

Dieses Naphthalin-Sulfiergemisch hatte formal folgende Zusammensetzung:

59,3 Gew.-% Naphthalin-1,5-Disulfonsäure

0,3 Gew.-% Naphthalin-1,3-Disulfonsäure

7,2 Gew.-% Naphthalin-1,6-Disulfonsäure

2,8 Gew.-% Naphthalin-1,7-Disulfonsäure

2,3 Gew.-% Naphthalin-1,3,5-Trisulfonsäure

4,8 Gew.-% Naphthalin-1,3,6-Trisulfonsäure

0,6 Gew.-% Naphthalin-1,3,7-Trisulfonsäure

22,8 Gew.-% $SO_3$

Gleichzeitig wurden 104 kg Mischsäure (34 Gew.-% $HNO_3$, 43 Gew.-% $SO_3$, 23 Gew.-% $H_2SO_4$) eindosiert. Die Reaktionsmischung wurde 2 Stunden bei 40 °C nachgerührt. In einem Fällungskessel wurden 365 kg Wasser bzw. Waschwasser aus der vorherigen Partie vorgelegt. Unter Rühren und Kühlen wurde die Nitriermischung bis maximal 85 °C einlaufen gelassen. Dann wurden 160 kg 30 Gew.-%ige Magnesiumsulfat-Lösung bei 85 °C zulaufen gelassen. Die Reaktionsmischung wurde unter Rühren auf 60 °C abgekühlt und das ausgefallene Produkt auf einem Pressfilterautomaten abfiltriert. Das Produkt wurde mit 560 kg 45 Gew.-%iger $H_2SO_4$ gewaschen, in 450 kg Wasser angeschlämmt, erneut filtriert und mit 250 kg Wasser gewaschen. Die Waschlauge aus der Anschlämmung und der Wasserwäsche wurden recyclisiert.

Es wurden 222 kg feuchtes Produkt mit folgender Zusammensetzung erhalten:

2-Nitronaphthalin-4,8-disulfonsäure-Magnesiumsalz (MG 355,3) 54,0 Gew.-%

2-Nitronaphthalin-4,7-disulfonsäure-Magnesiumsalz (MG 355,3) 0,2 Gew.-%

Naphthalin-1,5-disulfonsäure (MG 288,3) 0,2 Gew.-%

Schwefelsäure 0,7 Gew.-%

Wasser 44,7 Gew.-%

Die Ausbeute betrug bei Rückführung der Waschlaugen 67,5% bezogen auf Naphthalin, bzw. 87% bezogen auf 1,5-disulfiertes Naphthalin in Form des Naphthalinsulfierungsproduktes.

**Patentansprüche**

1. Verfahren zur Herstellung des Magnesiumsalzes von 2-Nitronaphthalin-4,8-disulfonsäure durch Nitrierung von 1,5-disulfiertem Naphthalin, Abtrennung von 2-Nitronaphthalin-4,8-disulfonsäure als Magnesiumsalz und Wäsche des Magnesiumsalzes, dadurch gekennzeichnet, dass man

a) von 1,5-disulfiertem Naphthalin in Form eines Gemisches ausgeht, das erhalten worden ist durch Zusammengabe von Naphthalin und $SO_3$ in Gegenwart eines inerten Lösungsmittels bei Temperaturen im Bereich von − 40 bis +20 °C, wobei das Verhältnis von zugegebenem $SO_3$ zu zugegebenem Naphthalin während der gesamten Zugabe im Bereich von 2,5 bis 5 Mol $SO_3$ Mol Naphthalin gelegen hat,

b) dieses Gemisch, gegebenenfalls nach Abtrennung des inerten Lösungsmittels, in wasserfreier Schwefelsäure mit einer Salpetersäure, Schwefelsäure und mindestens 0,7 Mol $SO_3$ pro Mol Salpetersäure enthaltenden Mischsäure bei Temperaturen im Bereich von 10 bis 60 °C und im Verlaufe von 1 bis 10 Stunden nitriert, wobei man das Gemisch und die Mischsäure simultan oder wechselweise in Portionen mit vorgelegter Schwefelsäure oder dem sich bildenden Reaktionsgemisch vereinigt.

c) danach zunächst Wasser und dann in Abwesenheit des inerten Lösungsmittels bei 60 bis 120 °C soviel einer Magnesiumionen abgebenden Verbindung zugibt, dass pro Mol 2-Nitronaphthalin-4,8-disulfonsäure 1,0 bis 1,3 Mol Magnesiumionen zugegeben werden und eine Schwefelsäure-Konzentration im Bereich von 40 bis 60 Gew.-% (bezogen auf Schwefelsäure und Wasser) eingehalten wird und

d) das ausgefallene Magnesiumsalz der 2-Nitronaphthalin-4,8-disulfonsäure bei einer Temperatur im Bereich von 30 bis 80 °C abtrennt und anschliessend mit maximal 60 gew-%iger Schwefelsäure und/oder Wasser wäscht.

2. Verfahren nach Anspruch 1, dadurch gekennzeichnet, dass man in Stufe b) ein Gemisch einsetzt, das erhalten worden ist durch Vereinigung von Naphthalin und $SO_3$, jeweils getrennt gelöst in Dichlormethan, bei − 10 bis − 5 °C, wobei während der gesamten Zugabe ein Molverhältnis von zugegebenem $SO_3$ zugegebenem Naphthalin im Bereich von 2,5 bis 3,6 eingehalten wurde.

3. Verfahren nach Anspruch 1 und 2, dadurch gekennzeichnet, dass man in Stufe b) zunächst das inerte Lösungsmittel abtrennt.

4. Verfahren nach Anspruch 1 bis 3, dadurch gekennzeichnet, dass man in Stufe b) pro Mol Naphthalin in Form des Gemisches, 1 bis 10 Mol 100%ige $H_2SO_4$ einsetzt.

5. Verfahren nach Anspruch 1 bis 4, dadurch gekennzeichnet, dass man in Stufe b) pro Mol Naphthalin in Form des Gemisches 1,0 bis 1,5 Mol Salpetersäure in Form der Mischsäure einsetzt.

6. Verfahren nach Anspruch 1 bis 5, dadurch gekennzeichnet, das die in Stufe b) eingesetzte Mischsäure 0,8 bis 1,2 Mol $SO_3$ pro Mol Salpetersäure enthält.

7. Verfahren nach Anspruch 1 bis 6, dadurch gekennzeichnet, dass in Stufe c) wässrige Magnesiumsulfat-Lösung bei 75 bis 100 °C zugegeben wird.

8. Verfahren nach Anspruch 1 bis 7, dadurch gekennzeichnet, das in Stufe d) das Magnesiumsalz der 2-Nitronaphthalin-4,8-disulfonsäure bei 40 bis 70 °C abgetrennt wird.

9. Verfahren nach Anspruch 1 bis 8, dadurch gekennzeichnet, dass in Stufe d) das Magnesiumsalz der 2-Nitronaphthalin-4,8-disulfonsäure auf einem Pressfilterautomaten abgetrennt wird.

10. Verfahren nach Anspruch 1 bis 9, dadurch gekennzeichnet, dass in Stufe d) das Magnesiumsalz der 2-Nitronaphthalin-4,8-disulfonsäure mit 0,5 bis 3 kg 40 bis 60%iger Schwefelsäure pro Mol des Magnesiumsalzes der 2-Nitronaphthalin-4,8-disulfonsäure gewaschen wird.

## Revendications

1. Procédé de préparation du sel de magnésium de l'acide 2-nitronaphtalène-4,8-disulfonique par nitration de naphtalène-1,5-disulfoné, par séparation de l'acide 2-nitronaphtalène-4,8-disulfonique sous forme de son sel de magnésium et par lavage du sel de magnésium, ce procédé étant caractérisé en ce que:

a) on part du naphtalène-1,5-disulfoné sous forme d'un mélange que l'on obtient en ajoutant conjointement du naphtalène et du $SO_3$ en présence d'un solvant inerte à des températures se situant dans l'intervalle compris entre $-40$ et $+20$ °C, le rapport entre le $SO_3$ et le naphtalène ajoutés se situant, au cours de toute l'addition, dans l'intervalle de 2,5 à 5 moles de $SO_3$ par mole de naphtalène,

b) éventuellement après séparation du solvant inerte, on soumet ce mélange dans de l'acide sulfurique anhydre à une nitration avec un acide mixte contenant de l'acide nitrique, de l'acide sulfurique et au moins 0,7 mole de $SO_3$ par mole d'acide nitrique, à des températures se situant dans l'intervalle de 10 à 60 °C et pendant une période de 1 à 10 heures, le mélange et l'acide mixte étant combinés simultanément ou alternativement par portions avec l'acide sulfurique préalablement déposé ou le mélange réactionnel qui se forme,

c) ensuite, on ajoute tout d'abord de l'eau puis, en absence du solvant inerte et à une température de 60 à 120 °C, on ajoute un composé cédant des ions magnésium en une quantité calculée de telle sorte que, par mole d'acide 2-nitronaphtalène-4,8-disulfonique, on ajoute 1 à 1,3 mole d'ions magnésium en maintenent une concentration en acide sulfurique se situant dans l'intervalle allant de 40 à 60% en poids (calculés sur l'acide sulfurique et l'eau),

d) on sépare le sel de magnésium précipité de l'acide 2-nitronaphtalène-4,8-disulfonique à une température se situant dans l'intervalle allant de 30 à 80 °C, puis on le lave avec de l'acide sulfurique en une concentration maximale de 60% en poids et/ou avec de l'eau.

2. Procédé suivant la revendication 1, caractérisé en ce que, lors de l'étape (b), on utilise un mélange obtenu par la combinaison de naphtalène et de $SO_3$, dissous chacun séparément dans du dichlorométhane, à une température de $-10$ à $-5$°C, en maintenant, pendant toute l'addition, un rapport molaire se situant dans l'intervalle de 2,5 à 3,6 entre le $SO_3$ et le naphtalène ajoutés.

3. Procédé suivant les revendications 1 et 2, caractérisé en ce que, lors de l'étape (b), on sépare tout d'abord le solvant inerte.

4. Procédé suivant les revendications 1 à 3, caractérisé en ce que, lors de l'étape (b), on utilise 1 à 10 moles de $H_2SO_4$ à 100% par mole de naphtalène sous forme du mélange.

5. Procédé suivant les revendications 1 à 4, caractérisé en ce que, lors de l'étape (b), on utilise 1 à 1,5 mole d'acide nitrique sous forme d'acide mixte par mole de naphtalène sous forme du mélange.

6. Procédé suivant les revendications 1 à 5, caractérisé en ce que l'acide mixte utilisé lors de l'étape (b) contient 0,8 á 1,2 mole de $SO_3$ par mole d'acide nitrique.

7. Procédé suivant les revendications 1 à 6, caractérisé en ce que, lors de l'étape (c), on ajoute une solution aqueuse de sulfate de magnésium à une température de 75 à 100 °C.

8. Procédé suivant les revendications 1 à 7, caractérisé en ce que, lors de l'étape (d), on sépare le sel de magnésium de l'acide 2-nitronaphtalène-4,8-disulfonique à une température de 40 à 70 °C.

9. Procédé suivant les revendications 1 à 8, caractérisé en ce que, lors de l'étape (d), on sépare le sel de magnésium de l'acide 2-nitronaphtalène-4,8-disulfonique au moyen d'un filtre-presse automatique.

10. Procédé suivant les revendications 1 à 9, caractérisé en ce que, lors de l'étape (d), on lave le sel de magnésium de l'acide 2-nitronaphtalène-4,8-disulfonique avec 0,5 à 3 kg d'acide sulfurique à 40–60% par mole du sel de magnésium de l'acide 2-nitronaphtalène-4,8-disulfonique.

## Claims

1. Process for the preparation of the magnesium salt of 2-nitronaphthalene-4,8-disulphonic acid by nitrating 1,5-disulphonated naphthalene, separating off 2-nitronaphthalene-4,8-disulphonic acid as the magnesium salt and washing the magnesium salt, characterised in that

a) the starting material used is 1,5-disulphonated naphthalene in the form of a mixture which has been obtained by bringing together naphthalene and $SO_3$ in the presence of an inert solvent at temperatures in the range from $-40$ to $+20$ °C, the ratio of $SO_3$ added to naphthalene added having been in the range from 2,5 to 5 mols of $SO_3$ per mol of naphthalene throughout the entire addition,

b) after separating off the inert solvent if appropriate, this mixture is nitrated in anhydrous sulphuric acid with a mixed acid containing nitric acid, suphuric acid and at least 0,7 mol of $SO_3$ per mol of nitric acid at temperatures in the range from 10 to 60 °C and in the course of 1 to 10 hours, the mixture and the mixed acid being combined simultaneously or alternately in portions with the sulphuric acid initially introduced or the reaction mixture formed,

c) thereafter, first water is added, and then a compound which supplies magnesium ions is added, in the absence of the inert solvent and at 60 to 120 °C, in an amount such that 1,0 to 1,3 mols of magnesium ions are added per mol of 2-nitronaphthalene-4,8-disulphonic acid and the sulphuric acid concentration is maintained in the range from 40 to 60% by weight (relative to sulphuric acid and water), and

d) the magnesium salt of 2-nitro-naphthalene-4,8-disulphonic acid which has precipitated is separated off at a temperature in the range from 30 to 80 °C and then washed with at most 60% strength by weight sulphuric acid and/or with water.

2. Process according to Claim 1, characterised in that in stage (b), a mixture is employed which has been obtained by combining naphthalene and $SO_3$, each dissolved separately in methylene chloride, at −10 to −5 °C, the molar ratio of $SO_3$ added to naphthalene added having been maintained in the range from 2,5 to 3,6 : 1 throughout the entire addition.

3. Process according to Claims 1 and 2, characterised in that, in stage b), the inert solvent is first separated off.

4. Process according to Claims 1 to 3, characterised in that 1 to 10 mols of 100% pure $H_2SO_4$ are employed in stage b) per mol of naphthalene in the form of the mixture.

5. Process according to Claims 1 to 4, characterised in that 1,0 to 1,5 mols of nitric acid in the form of the mixed acid are employed in stage b) per mol of naphthalene in the form of the mixture.

6. Process according to Claims 1 to 5, characterised in that the mixed acid employed in stage b) contains 0,8 to 1,2 mols of $SO_3$ per mol of nitric acid.

7. Process according to Claims 1 to 6, characterised in that, in stage c), aqueous magnesium sulphate solution is added at 75 to 100 °C.

8. Process according to Claims 1 to 7, characterised in that, in stage d), the magnesium salt of 2-nitronaphthalene-4,8-disulphonic acid is separated off at 40 to 70 °C.

9. Process according to Claims 1 to 8, characterised in that, in stage d), the magnesium salt of 2-nitro-naphthalene-4,8-disulphonic acid is separated off on a filter press.

10. Process according to Calims 1 to 9, characterised in that, in stage d), the magnesium salt of 2-nitronaphthalene-4,8-disulphonic acid is washed with 0,5 to 3 kg of 40 to 60% strength sulphuric acid per mol of the magnesium salt of 2-nitronaphthalene-4,8-disulphonic acid.